# EUROPEAN PATENT SPECIFICATION

(11) **EP 3 013 360 B1**
(45) Date of publication and mention of the grant of the patent: **15.11.2017**
(21) Application number: 14734540.9
(22) Date of filing: 09.06.2014
(51) Int. Cl.: A61K 38/47, C12N 9/46

(54) **ANTI-MICROBIAL AGENTS AND USES THEREFOR**
ANTIMIKROBIELLE MITTEL UND VERWENDUNGEN DAVON
AGENTS ANTIMICROBIENS ET LEURS UTILISATIONS

(30) Priority: 25.06.2013 GB 201311272
(43) Date of publication of application: 04.05.2016
(73) Proprietor: UCL Business PLC, Greater London, W1T 4TP (GB)
(72) Inventor: ALLAN, Elaine, London Greater London WC1X 8LD (GB)
(74) Representative: Kremer, Simon Mark
(86) International application number: PCT/GB2014/051766
(87) International publication number: WO 2014/207435

(56) References cited:
- WO-A1-01/04270
- WO-A1-98/00529
- WO-A1-2006/034710
- WO-A2-03/062409
- GB-A- 2 261 877
- US-A1- 2008 031 998

## Description

### Technical field

The present invention relates generally to methods and materials for use in treating or controlling *Campylobacter* infection or colonisation.

### Background art

*Campylobacter* infection is a major global health problem in both the developing and developed world (Allos, 2001; Coker et al., 2002; Janssen et al., 2008). There are 1.3 million cases in the US annually and the incidence in 2012 had increased by 14% compared with 2006-2008 (CDC, 2013). In Europe, there were 220,209 reported cases in 2011, a 2.2% rise compared to 2010 (European Food Safety Authority, 2013). Disease outcomes in humans range from mild, non-inflammatory, self-limiting diarrhea to prolonged, inflammatory diarrhea and occasionally more serious extraintestinal complications, such as Guillain-Barre syndrome and reactive arthritis (Allos, 2001). Ninety percent of human disease is attributed to *C. jejuni,* while *Campylobacter coli* accounts for the remainder (Gillespie et al., 2002). In contrast to human infection, *C. jejuni* establishes a persistent but largely asymptomatic infection in animals and birds, and the major risk of human infection is through the handling and consumption of poultry meat (http://www.food.gov.uk/safereating/foodchain/summary/; Wilson 2008; Agedbola et al., 1990; Humphrey et al., 2007; Garin et al., 2012).

*Campylobacters* are commensals of, primarily, the lower gastrointestinal (GI) tract of the chicken and contamination of the flesh of the bird as well as the environment occurs as a result of the spillage of intestinal contents during slaughter (FAO/WHO report, 2009). Within the GI tract of chickens, *Campylobacter* spp. reside in densely-packed groups of cells apparently surrounded by mucus within the luminal crypts (Beery et al., 1988). Of particular note is that the bacteria are adherent to the mucus, and not directly to the epithelial surface (Beery et al., 1988). This arrangement is reminiscent of a biofilm. Microcolonies and biofilms have been described on experimentally-infected human ileum ex vivo suggesting that this is also the mode of growth during human infection (Haddock et al., 2010; Edwards et al., 2010).

In the laboratory, three distinct types of biofilm were described by Joshua et al (2006): a surface-attached biofilm, a pellicle at the liquid-gas interface, and an unattached aggregate or floc. Monospecies or multispecies biofilms form on materials found in animal production watering systems and other materials used in industrial facilities, such as stainless steel and polyvinyl chloride (Joshua et al 2006, Buswell, et al 1998, Sanders et al., 2007, Sanders et al., 2008, Trachoo et al., 2002, Reeser et al., 2007, Reuter et al., 2010, Kalmokoff et al., 2006), and these biofilms may represent a persistent source of infection for the animals as well as for humans working in these facilities (Zimmer et al., 2003, Trachoo et al., 2002, de Perio et al., 2013).

Despite a number of papers describing biofilm formation by *C. jejuni* (Murphy et al., 2006, Reeser et al., 2007, Joshua et al., 2006, Buswell, et al., 1998, Sanders et al., 2007,

Sanders et al., 2008, Trachoo et al., 2002, Reeser et al., 2007, Reuter et al., 2010, Kalmokoff et al., 2006) few have addressed the nature of the extracellular polymeric matrix (EPM), a defining feature of a biofilm.

The most studied component of the biofilm EPM is polysaccharide. *C. jejuni* produces several surface-associated carbohydrate structures including lipooligosaccharide, capsular polysaccharide and both N- and 0-linked glycoproteins (Guerry and Szymanski 2008) but there is no evidence to suggest that any of these glycogonjugates contribute to biofilm formation. In the study by Joshua et al., strains containing mutations in genes *kpsM, neuB1* and *pglH,* deficient for synthesis of capsular polysaccharide, lipooligosaccharide and N-linked glycoproteins, respectively, showed no reduction in biofilm formation; indeed, the *neuB1* and *kpsM* mutants formed larger pellicles than the wild-type strain, suggesting that the presence of these glycoconjugates is inhibitory for biofilm formation, a finding confirmed for the *kpsM* mutant by McLennan et al (2008). A surface polysaccharide reactive with calcofluor white, indicative of beta-1,3 and/or beta-1,4-glycosidic bonds, was reported by McLennan et al (2008) and up-regulation of this polysaccharide was associated with increased biofilm formation, suggesting that this polysaccharide could be a component of the EPM. This study also showed that a mutant in the gene *gne,* which encodes a bifunctional UDP-GlcNAc/Glc4 epimerase required for biosynthesis of CPS, LOS, and *N*-linked carbohydrates (Bernatchez et al., 2005), produced a biofilm that was no different from the wild-type strain, indicating that the biofilm phenotype is independent of the known glycan structures (McLennan et al., 2008).

In a study by Moe et al., (2010), EPM material was described that stained with ruthenium red, suggestive of a polyanionic structure, however, no structural data was provided. A second phospholipid-anchored polysaccharide that is independent of *kpsM*was described in strain 81-176 although no structural data was provided (Bacon et al., 2001). A surface-located alpha-1,4- glucan was subsequently described by Papp-Szabó et al., (2005) who suggested that this is the second CPS described by Bacon et al, however, no functional information is available for this polysaccharide.

Eradication of *Campylobacterfrom* poultry flocks is considered the most effective strategy for reducing the bacterium in the food chain and therefore preventing human infection.

US2006/0083731 relates to Xylanase or a cellulase for the manufacture of an agent for the treatment and/or prophylaxis of bacterial infection in an animal caused by *Salmonella, Campylobacter* or *Clostridium perfringens.* In preferred forms the xylanase is used in combination with wheat to form an animal feed.

WO 1993/001800 discloses the use of a protease for the preparation of a medicament effective against intestinal pathogens in animals.

EP-A-0 681 787 discloses use of a carbohydrase or protease for the manufacture of an agent for the treatment of *Coccidiosis.*

One method suggested in the art is the use of bacteriophages (see Connerton et al., 2011). Such a strategy, even if successful, would require a large number of phages to cover all the different strains of bacteria, and would also be inherently susceptible to resistance developing.

Another method suggested in the art is vaccination (Layton et al., 2011). However there are significant challenges to this since it requires the identification of a suitable target antigen which is immunologically active in the gut of the animal. Despite some success with a live *Salmonella* vaccine engineered to express *Campylobacterantigens* (Layton et al., 2011), the use of a genetically engineered (GM) vaccine in food production raises issues of consumer acceptability and commercial viability.

Thus it can be seen that novel methods for controlling *Campylobacter* would provide a contribution to the art.

### Disclosure of the invention

The present inventors have found that the *Campylobacter* EPM is produced when the bacterium is in contact with a host molecule, with one specific signal being pancreatic alpha-amylase. They showed that exposure to this signal resulted in a 100% increase in biofilm formation in vitro. The EPM was shown to increase adhesion of the bacteria to intestinal epithelial cells. It was further demonstrated that exposing the bacterium to pancreatic amylase before inoculation into chickens resulted in a highly significant increase in colonisation, presumably as a result of an increased ability to form a biofilm in the intestine of the chicken.

The key polysaccharide components of the *Campylobacter* EPM have not been previously been characterised. However the present inventors have now shown one such key component of the *Campylobacter* biofilm is alpha-dextran. Furthermore the inventors have shown that commercially-available dextranase enzymes can disrupt the biofilm.

Thus different aspects of the invention employ one or more enzymes which hydrolyse or otherwise degrade or inhibit the formation of dextran in order to disrupt the formation or stability of the biofilm, for example to inhibit *Campylobactergrowth* or colonization, or treat *Campylobacter* infection in an animal. Preferred enzymes are dextranases, although other enzymes which hydrolyse or otherwise degrade or inhibit the formation of dextran may be employed *mutatis mutandis* in the aspects and embodiments of the invention described herein.

In one aspect of the invention there is provided a composition comprising dextranase for use in method for inhibiting colonization of an animal by *Campylobacter,* said method comprising the steps of administering orally to said animal the composition comprising the dextranase.

"Colonization" in this context will be understood to refer to the GI tract of the animal.

Such methods can be used with the purpose of eradicating or reducing the prevalence of *Campylobacter* in a population of animals, by feeding the population with a feed comprising the composition.

In preferred embodiments the animal is a poultry animal, such as a chicken. However the invention may also be applied to other livestock which are reared for human consumption e.g. cattle and pigs.

In one embodiment, the composition is administered to new-born chicks, ranging in age from about 1 to about 7 days post hatching; in another embodiment the composition is administered after 1 week but within 2, 3, or 4 weeks post hatching; in another embodiment the composition is administered after 2 or 3 e.g. after decline of the maternal antibodies.

Also described herein is a method of reducing the incidence of *Campylobacter* contamination in a poultry-containing human foodstuff, the method comprising reducing colonisation of the poultry flock from which the human foodstuff is derived by feeding the flock a composition comprising dextranase.

Also described herein is a method of producing a poultry-containing foodstuff having a reduced probability of *Campylobacter* contamination, the method comprising:
(i) reducing *Campylobacter* colonisation of a poultry flock by feeding the flock a composition comprising dextranase;
(ii) producing the foodstuff from said flock.

In one aspect of the invention there is provided a method of inhibiting the virulence or growth, or reducing the number, of *Campylobacter* bacteria in an environment, said method comprising the step of contacting the bacteria or the environment with a composition comprising dextranase. Said method is performed ex vivo.

It will be appreciated that "inhibiting" in this and other aspects of the invention may lead to 'preventing' but that is not a requirement, and "inhibiting" does not circumscribe complete success - rather it indicates a reduction compared to a reference point in which the dextranase is not used.

In one aspect of the invention there is provided a method of disrupting a *Campylobacter* bacterial biofilm, said method comprising the step of contacting the biofilm with a composition comprising dextranase. Said method is performed ex vivo.

In one aspect of the invention there is provided a method of inhibiting the formation of a *Campylobacter* bacterial biofilm, said method comprising the step of contacting the bacteria with a composition comprising dextranase. Said method is performed ex vivo.

Described herein is a method of treating *Campylobacter* infection in an animal, said method comprising the step of feeding the animal a composition comprising dextranase. Said infection may be one which is symptomatic or asymptomatic.

In one aspect there is a provided a composition comprising dextranase for use in any of the methods described herein, particularly those methods which are therapeutic methods practised on the animal body.

Some particular aspects and embodiments of the invention will now be discussed in more detail:

A biofilm in the present context is surface-associated community of bacteria surrounded by a hydrated extracellular polymeric matrix (EPM) the most studied component of which is polysaccharide, but may also include proteins, nucleic acids, and lipids (Donlan et al.,2002; Davey and O'Toole, 2000). Within the GI tract of chickens, *Campylobacter* exists as densely-packed groups of cells surrounded by mucus which indicates that they are in biofilms. As demonstrated in the Examples herein, the dextran comprising biofilms are highly relevant to the ability of *Campylobacterto* colonise the GI tract.

A number of Campylobacter strains are known in the art, and these may be treated using the methods of the present invention.

Preferred Campylobacter target strains are those which cause diarrheal disease e.g. C. jejuni and C.coli. Strains can be distinguished by methods known in the art (see e.g. Patton et al JOURNAL OF CLINICAL MICROBIOLOGY, Apr. 1991, p. 680-688).

Alpha-dextran is a polymer of glucose linked by alpha-1,6 glycosidic bonds:

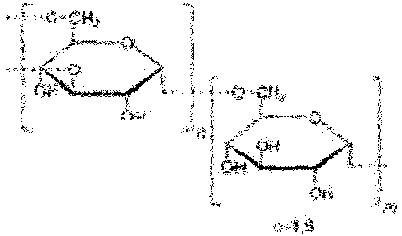

Dextranases are α-1, 6-glucanases (E.C. 3.2.1.11), also known as 1, 6-α-D-glucan 6-glucanohydrolases, which degrade the α-1, 6-glycosidic linkages in dextran. Several micro-organisms are known to be capable of producing dextranases, among them fungi of the genera Penicillium, Paecilomyces, Aspergillus, Fusarium, Spicaria, Verticillium, Helminthosporium and Chaetomium; bacteria of the genera Lactobacillus, Streptococcus, Cellvibrio, Cytophaga, Brevibacterium, Pseudomonas, Corynebacterium, Arthrobacter and Flavobacterium, and yeasts such as Lipomyces starkey (see e.g. WO1998000529).

Dextranases are produced for use in sugar beet processing and beer fermentation. Dextranases have also been used to disrupt the dental plaque biofilm (see EP1011700).

Dextranases are available commercially from numerous sources at either as a fine chemical or a bulk commodity e.g. Sigma-Aldrich, Worthington Biochemical Corporation, Gaocheng Baoli Plastic Products Co., Ltd, China, VARUNA BIOCELL PRIVATE LIMITED, India. Etc. An example of a commercially available dextranase, sold as an industrial enzyme for breaking down raw sugar juice, is Dextranase 50L from Novo Nordisk produced by fermentation of a strain of Paecilomyces sp..

It will be apparent that dextranases for use in the present invention may thus be selected from those known in the art in the light of the present disclosure according to the required application, and the choice of dextranase does not *per se* form a part of the present invention.

In a preferred embodiment of the invention the dextranase is a recombinant enzyme.

In a preferred embodiment of the invention the dextranase is a fungally derived enzyme.

In aspects of the invention relating to feeding of compositions to animals to inhibit colonisation or the like, the dextranase should retain activity at the temperatures and pHs prevailing in the digestive tract of animals i.e. typically between 20°C and 45°C, especially around 42°C. Furthermore the enzyme should be protected from the digestion process in order to reach the target site (small intestine) at effective concentrations.

Thus is may be preferred that the enzyme is encapsulated or otherwise provided in protected form in the composition.

Processes for protecting enzymes so that they can retain activity in the target site are known in the art. Some known technologies are discussed in "NEXT GENERATION THERMO-TOLERANT ENZYMES FOR REDUCING FEEDING COSTS" Bedford (2008) 16th Annual ASA-IM SEA Feed Technology and Nutrition Workshop, May 26-30, 2008, The Regent, Singapore. These include use of an encapsulating 'coat'. Commonly used encapsulation systems applicable to enzymes or enzyme microgranules include dripping, spraying, emulsion coating, spray coating, and suspension coating. Known systems include those described in WO1988/001512; WO1998/014601; WO2007/072535; WO1985/005288. Companies specialising in enzyme protection (e.g. phytase protection) for the feed industries include AB Vista, Adisseo, Azelism, BASF, Danisco (Genencor-DuPont), Frank Wright Trouw Nutrition International, HUVEPHARMA, Kiotechagil, Kemin and Optivite. Analogous methods or compositions to those known in the art may be used to protect the dextranase employed in the present invention.

Thus a protected composition may be one in which the dextranase is present in immobilised form in the core of an enzyme microgranule, wherein the core may be encapsulated within a water soluble film, and coated with an enteric coating comprising an alkali soluble, acid insoluble polymer, or a high molecular weight polymer whose structure is substituted with or contains windows of fatty acid or other material capable of being solubilized by intestinal juices (see WO1988/001512).

In other embodiments the composition may comprise (i) granules comprising the enzyme in association with a weak base and partially coated with a delayed release material soluble in intestinal juice; (ii) an acidifying agent having a pH between about 1.5 to about 6 when in solution; and (iii) a gel forming agent (see WO1993001800).

Those skilled in the art will appreciate that the precise manner in which the enzyme is protected may be selected from those known in the art in the light of the present disclosure according to the required application, and does not *per se* form a part of the present invention.

In the practise of the invention described herein, the composition may be administered in combination with feed for said animal, or as part of the feed itself (e.g. as an intimately mixed mixture with nutritional components such as a cereal e.g. wheat).

By way of non-limiting example, where the animal is a chicken, the dextranase may be fed to the chickens in an amount of about 0.0001 to about 10 grams of dextranase per kg of the feed, or 0.001 to about 1 gram of dextranase per kg of the feed, or 0.01 to about 0.1 gram of dextranase per kg of the feed.

This diet is preferably fed to the chickens without a withdrawal period prior to slaughtering of the chickens, such as to minimise the likelihood of opportunistic infection.

Preferably said diet does not contain an antimicrobial drug. However where said diet contains an antimicrobial drug, this may be at a concentration that is not effective for treatment and/or prophylaxis of bacterial infection in chickens caused by *Campylobacter* in the absence of the dextranase.

In other embodiments the composition may be is administered in the drinking water for said animal. Example concentrations may be an amount equivalent to an enzyme activity, calculated as enzyme activity units in the drinking water, in the range from 0.001 U to 1000 U/ml, preferably from 0.01 U/ml to 500 U/ml, especially from 0.1 U/ml to 100 U/ml.

Any sub-titles herein are included for convenience only, and are not to be construed as limiting the disclosure in any way.

The invention will now be further described with reference to the following non-limiting Figures and Examples. Other embodiments of the invention will occur to those skilled in the art in the light of these.

### Figures

Fig 1: the graph shows the increase in biofilm density in two different strains of *C. jejuni* following dextran induction by alpha-amylase. The increases are highly statistically significant ***p<0.0001.
Fig 2: the graph shows biofilm density of *C. jejuni* 11168H with 0.05 M Tris added (negative control) and with the addition of chicken pancreatic extract. The increase is highly statistically significant *** p<0.0001. This data shows that the bacterium is probably expressing the dextran when present in chicken intestine.
Fig 3: the graph shows the number of bacteria per gram of ileal contents in birds 7 d post infection. Bars indicate significant difference. There is a significant increase in colonisation when *C. jejuni* 11168H was grown with (+) compared to without (-) amylase (p=0.005). In contrast, there is no increase in colonisation of the *cj0511* mutant with pre-exposure to amylase. The wild type strain (11168H) was also present in significantly higher numbers in the ileum compared to the *cj0511* mutant (p=0.03).
Fig 4: the graph shows disruption of a preformed *C. jejuni* 11168H biofilm by commercially available fungal dextranase. The decrease in biofilm density after treatment with dextranase is highly statistically significant **p=0.001.

### Examples

### EXPERIMENTAL PROCEDURES

### Growth conditions

*C. jejuni* was stored at -70°C in Brucella broth (Oxoid) containing 15% glycerol and was grown on Columbia blood agar (CBA; Oxoid) containing 7% defibrinated horse blood (E & O Laboratories) or on Mueller Hinton agar (MHA; Oxoid) in a microaerobic atmosphere generated using Campypaks (Oxoid) in gas jars at 37°C. Charcoal (0.4%) was added to MHA (CMHA) to improve contrast for photography. Kanamycin (Km; 40 µg/ml) and chloramphenicol (Cm; 20 µg/ml) were used for selection as required. Eagle's Minimal Essential Medium-alpha (MEM-alpha; Sigma-Aldrich) or Standard American Petroleum Institute medium (SAPI; Miller et al., 2008) without glucose supplemented with 1.5% (w/v) agar were used as minimal media. Hog pancreatic alpha-amylase (HP amylase) and α-amylase from *Aspergillus oryzae were* purchased from Sigma-Aldrich and were used at concentrations of 15 nM, 100 nM and 100 µM.

### EPM purification and characterisation

Growth was removed from 4 MHA plates with or without 100 nM HP amylase and suspended in 5 ml phosphate buffered saline (PBS; Sigma-Aldrich). For proteomics, 1 µl of a 1/10 dilution of protease inhibitor cocktail was added (Sigma-Aldrich). The suspension was washed at 200 rpm on a rotary platform for 1.5 h at 30°C then vortexed for 15 min and further washed at 200 rpm for 1.5 h at 20°C. Bacteria were removed by centrifugation at 1,800 x g for 15 min and the supernatant was precipitated by addition of 4 volumes of ice cold acetone and incubation at 4°C overnight. The precipitate was recovered by centrifugation at 450 x g for 5 min, washed in distilled water, dried in a Speedvac SPD1010 (Thermo Scientific), dissolved in 1 ml of distilled water and stored at -70°C. Total carbohydrate was measured using the phenol-sulphuric acid assay (Dubois et al., 1956) with glucose as standard. Protein content was determined using a Micro BCA Protein Assay kit (Pierce, Thermo Scientific) according to the manufacturer's instructions. Further characterisation of the EPM carbohydrate was performed following dialysis (14 kDa MWCO) for 72 h at 4°C, against three changes of distilled water per day. The EPM was freeze dried and characterized using NMR spectroscopy (Laws et al., 2008), monomer analysis (Gerwig et al., 1978) and linkage analysis (Stellner et al., 1973). For comparison, the same experiments were performed with a commercial α-dextran standard (Sigma-Aldrich). Detailed experimental procedures have been reported elsewhere (Laws et al., 2008).

For proteomics, EPM samples were normalised by dry weight, separated by SDS-PAGE (10% acrylamide) and gels stained with Colloidal Coomassie brilliant blue (Sigma-Aldrich). Proteins were digested in-gel with trypsin and analysed by LC-MS/MS essentially as previously described (Khandavilli et al., 2008). MS data were used to interrogate the *C. jejuni* 81-176 genome database using Bioworks version 3.2 (Thermo Electron).

### Construction of a spoT mutant

A *spoT* mutant of *C. jejuni* 11168H was constructed by insertion of a kanamycin resistance gene, *aphA-3,* into a unique *Bg*/II site at nucleotide 624. A 1.2 kb fragment of *spoT* was amplified by PCR and cloned into pGEM-T-easy, generating pWJ1. A kanamycin resistance gene (*aphA3*) was inserted into the unique BgIII site in *spoT* generating pWJ2 which was electroporated into *C. jejuni* (van Vliet et al., 1998) selecting transformants on agar containing Km. The chromosomal insertion of *aphA3* was confirmed by PCR using primers annealing in *spoT* and *aphA3,* respectively.

### Genetic complementation of the cj0511 mutant

A complemented strain was constructed by insertion of *cj0511* under the control of the iron-inducible *fdxA* promoter (P_{fdxA}) into a pseudogene (*cj0046*) in the *cj0511* mutant as described by Gaskin et al., (2007). The 1.3 kb *cj0511* gene was PCR amplified from *C*. *jejuni* 11168H and cloned into pCfdxA (van Vliet, unpublished), selecting *E. coli* transformants on LB agar with 20 µg/ml Cm. A plasmid (pWJ4) with *cj0511* in the same orientation as P*_{fdxA}* was selected by PCR, and electroporated into the *cj0511* mutant, selecting transformants with Cm and Km. Insertion of *cj0511* within *cj0046* in the mutant chromosome was confirmed by PCR.

### Production of recombinant Cj0511

The *cj0511* coding sequence was PCR amplified from strain 11168H, cloned into pET151/D-TOPO (Invitrogen) to generate pWJ4 and transformed into *E. coli* BL21 Star (DE3). Histidine-tagged recombinant protein was purified using Ni-nitrilotriacetic acid agarose (Qiagen) according to the manufacturer's instructions. If not used immediately, the protein was stored at -70°C in 10% (v/v) glycerol.

### Autoagglutination and biofilm assays

Bacteria were harvested from MHA with or without 100 nM HP amylase (or chicken pancreatic extract) and suspended in 1 ml of Brucella broth (Becton Dickinson) to measure autoagglutination by the decrease in OD₆₀₀ at room temperature in air (Misawa and Blaser, 2000). For biofilm formation, a single colony was inoculated into Brucella broth and grown with shaking (50 rpm) for 16 h at 37°C in 5% CO₂, diluted to OD₆₀₀ of 0.1 in Brucella broth, with or without 100 nM HP amylase (or chicken pancreatic extract), and added to a borosilicate glass tube (VWR International). After 48 h at 37°C in 5% CO₂, the culture was decanted, the biofilms were washed with water, dried and stained for 5 min with 0.1 % (w/v) crystal violet (CV; Sigma-Aldrich) then washed again with water and dried. Bound CV was dissolved in 80% ethanol-20% acetone and OD₆₀₀ measured.

### Confocal laser scanning microscopy of biofilm

An overnight culture was prepared as described above, diluted to OD₆₀₀ of 0.1 in Brucella broth with or without 100 nM HP amylase and added to the wells of a 6-well tissue culture plate (Sarstedt). Two glass coverslips were immersed upright in each well and incubated for 48 h at 37°C in 5% CO₂. The coverslips were washed in PBS, stained with Live/Dead BacLight stain (Invitrogen) according to the manufacturer's instructions and visualised with a Bio-Rad confocal microscope attached to an Olympus BX51 upright microscope. Digital images were produced using Image J software (National Institutes of Health).

### Preparation of extract of chicken pancreas

A crude extract was prepared from 20 chicken pancreases using methods from Madhusudhan et al., (1987). The pancreases, stored for 4 months at -70°C were partially thawed, 1 ml of buffer was added (0.05 M Tris, 0.9% NaCl, 0.05 M CaCl₂, pH 8.0) and they were homogenised using a Ultra-Turrax T8 (IKA Labortechnik) tissue homogenizer. After centrifugation (1,000 x g, 20 min), the supernatant was recovered and the pH adjusted to 8.0 with NaOH. Amylase activity was quantified using a starch iodine assay (see above) with hog pancreatic amylase (Sigma-Aldrich) as standard.

### Resistance to environmental stress

The growth from 48 h MHA plates with and without 100 nM HP amylase was recovered in PBS (4°C) or MH broth (20°C in air) to OD₆₀₀ of 2.0. Samples were removed at 2 day intervals for viable counts on CBA plates. For survival at 65°C, bacteria were suspended in 1.0 ml of MH broth to OD₆₀₀ of 2.0, and counts were performed every 3 min. For survival at low pH, bacteria were suspended to OD₆₀₀ of 2.0 in PBS (adjusted to pH 4.0 with HCl) at 37°C and counts were performed every 15 min.

### Adhesion and invasion of Caco-2 cells

Human colon cancer cells (Caco-2) were cultured as previously described (Mills *et al.,* 2012). To a confluent monolayer of - 10⁶ Caco-2 cells, 10⁸ bacteria were added and incubated at 37°C in 5% CO₂ for 3, 6 and 24 h. To determine the number of interacting bacteria, the monolayers were washed three times with PBS and lysed by adding 0.2% (v/v) Triton X-100. Viable counts were performed by plating dilutions on CBA. To determine the number of invading bacteria, the monolayers were incubated with DMEM containing 150 µg/ml gentamicin for 2 h, washed three times with PBS, lysed by adding 0.2% (v/v) Triton X-100, and counts performed.

### Interaction with T84 human colonic epithelial cells

T84 cells were grown in complete DMEM/F12 HAM media for > 14 days in rat collagen pre-coated transwell dishes until the transepithelial electrical resistance (TEER) measurement was > 800 Ω confirming formation of tight-junctions in the monolayer. Coculture studies were performed at a multiplicity of infection of 10 in DMEM/F12 HAM + 10% (v/v) foetal calf serum for 6 or 24 h. Bacterial counts were performed by plating on CBA. IL-8 levels were determined by ELISA (eBioscience) from the supernatant of the apical surface at 24 h post-infection. TEER measurements were conducted at 24 h post-infection.

### Galleria mellonella infection model

*Galleria* larvae (Cornish Crispa Co.) were maintained on wood chips at 11 °C. *C. jejuni* 11168H was grown on MHA with or without 100 nM HP amylase and harvested in PBS. The infections were carried out as described by Champion et al. (2010).

### Infection of chickens

Day old broiler chickens were obtained from a commercial supplier and housed in biosecure housing. The study was performed under UK Home Office licence and approval by the local ethical review committee. Birds were fed a commercial diet, had access to food and water and had a gradual increase and decrease in light at the beginning and end of each day, as part of a 12 h light/darkness cycle. When the birds were 21 days old, groups of 30 were infected by oral gavage with 10⁵ of *C. jejuni* 11168H or the *cj0511* mutant grown on MHA with or without 100 µM HP amylase and resuspended in MH broth. Fifteen birds were euthanased at 4 and 7 days post infection and ileum and liver samples removed for culture. Ileum samples were serially diluted and plated on modified charcoal cefoperazone deoxycholate agar (mCCDA; Oxoid) incubated at 37°C for 48 h in microaerobic conditions. Liver samples were homogenised and enriched in modified Exeter broth incubated with minimal headspace at 37°C for 48 h and plated on mCCDA. Differences in the number of birds colonised were assessed using a Chi-square test. Differences in the number of bacteria found at each site were analysed using a Kruskal-Wallis test with Dunn's multiple comparison test.

### Disruption of a C. jejuni biofilm with fungal dextranase

Biofilms were grown on glass coverslips as described above for CLSM. To test for biofilm disruption, 200 µl of a 10 mg/ml suspension of Penicillum dextranase (Sigma) or the same volume of water was dispensed on to the biofilm and incubated for 2 h at 37°C. The biofilm was washed with PBS and the biomass quantified by CV staining as described above.

### Plate assay for dextranase activity

Bacteria were inoculated as lawns to MHA and grown in 5% CO₂ for 24 h. A well was cut in the centre of the bacterial lawn to which was added 70 µl of a 40 mg/ml suspension of Penicillum dextranase (Sigma). The plates were incubated in 5% CO₂ for 48 h and examined for the presence of a zone around the dextranase-containing well, indicative of dextranase activity.

### Example 1- physioloaical concentrations of pancreatic alpha-amylase is the signal for induction of EPM by the bacterium

As colonies of *C. jejuni* freshly isolated from stool are invariably more mucoid than laboratory strains, we hypothesised that the EPM is induced in *C. jejuni* in response to host molecules and that its expression is lost upon laboratory culture. *C. jejuni* is known to respond to the presence of host-specific signals including a low oxygen environment, bile salts, norepinephrine (Mills et al., 2012, Malik-Kale et al., 2008, Cogan et al., 2007).

We demonstrated that the bacterium detects and responds to the presence of host pancreatic enzymes. Detection of the alpha-amylase signal requires an intact *cj0511* gene, encoding a predicted secreted protease. Previous studies reported the Cj0511 protein in the secretome or specifically within outer membrane vesicles (Prokhorova et al., 2005, Elmi et al 2012) and we demonstrated for the first time that it is a protease capable of degrading pancreatic alpha-amylase.

### Example 2 - the secreted carbohydrate is alpha-dextran

In this example we show that exposure to pancreatic alpha-amylase from mammals or birds results in secretion of an alpha-dextran that promotes biofilm formation in vitro.

### Growth in the presence of mammalian pancreatic alpha-amylase results in a large mucoid colony

Physiological concentrations of mammalian pancreatic alpha-amylase are estimated to be nanomolar (Slaughter et al., 2001). Hog pancreatic alpha-amylase (HP amylase) was incorporated into MHA agar (15 nM, 100 nM and 100 µM) and formation of large mucoid colonies of *C. jejuni* 11168H was observed only at the highest concentration. Mucoid colonies were also produced by other *C. jejuni* strains (81-176, 81116, G1, X) in response to 100 µM HP amylase but not by *Campylobacterrectus* NCTC 11489 (data not shown). Culture on 100 µM microbial alpha-amylase (from *Aspergillus oryzae*) did not result in mucoid colonies in any of the strains tested (data not shown).

### Mucoidy in fresh clinical isolates is the result of exposure to pancreatic amylase

Since *C. jejuni* isolates from human stools are more mucoid than laboratory strains (Allan, personal observation), we hypothesised that this was due to recent exposure to pancreatic alpha-amylase. Two recent clinical isolates were sub-cultured on MHA until mucoidy was lost: three sub-cultures were required in both strains. The non-mucoid colonies were then plated on to MHA containing 100 µM HP amylase and mucoidy was restored. This shows that the mucoid phenotype is physiologically relevant, is rapidly lost on culture and is the result of exposure to pancreatic alpha-amylase.

### Mucoid colonies secrete increased amounts of carbohydrate and protein

As a mucoid colony is suggestive of exopolymer secretion, the EPM was prepared from colonies grown in the presence of increasing concentrations of HP amylase by gentle washing in PBS. The recovered material was concentrated by precipitation and the carbohydrate content determined by phenol-sulphuric acid assay. The data showed that there is an approximately 2 fold increase in carbohydrate secretion at physiological concentrations of alpha-amylase (100 nM) and above.

A *kpsM* mutant, which is unable to export CPS (Karlyshev et al., 2000), and *waaC* and *waaF* mutants, lacking the heptosyltransferases responsible for adding heptoses to the core oligosaccharide of LOS (Kanipes et al., 2006), also showed increased carbohydrate secretion of comparable magnitude to the wild-type in the presence of amylase. Since mutation of *spoT,* encoding a regulator of the stringent response, was reported to overproduce a CFW-reactive polysaccharide which promotes biofilms (McLennan et al., 2008), we constructed this mutant in strain 11168H but found that it secreted levels of carbohydrate similar to the wild-type strain in both the presence and absence of amylase. Collectively these data show that the secreted polysaccharide is independent of previously described surface glycans.

### The secreted carbohydrate is alpha-dextran

The EPM recovered from *C. jejuni* 11168H and the *kpsM* mutant, together with an alpha-dextran standard, provided ¹H and ¹³C-NMR which were identical and a series of 2D-NMR (COSY, HSQC & HMBC) were also recorded (data not shown) to confirm the identity of the exopolysaccharide as an alpha-dextran. Monomer analysis confirmed that glucose was the only monosaccharide present and linkage analysis showed exclusively α-1,6-glycosidic links

### Secretion of EPM promotes autoagglutination and biofilm formation in vitro

Since autoagglutination is likely to be a prerequisite for micro-colony formation, this phenotype was measured in the presence and absence of 100 nM HP amylase The data shows that growth of both 11168H and 81-176 in the presence of alpha-amylase resulted in increased autoagglutination assessed by the decrease in optical density over time as the suspended bacteria agglutinate and precipitate at the bottom of a cuvette.

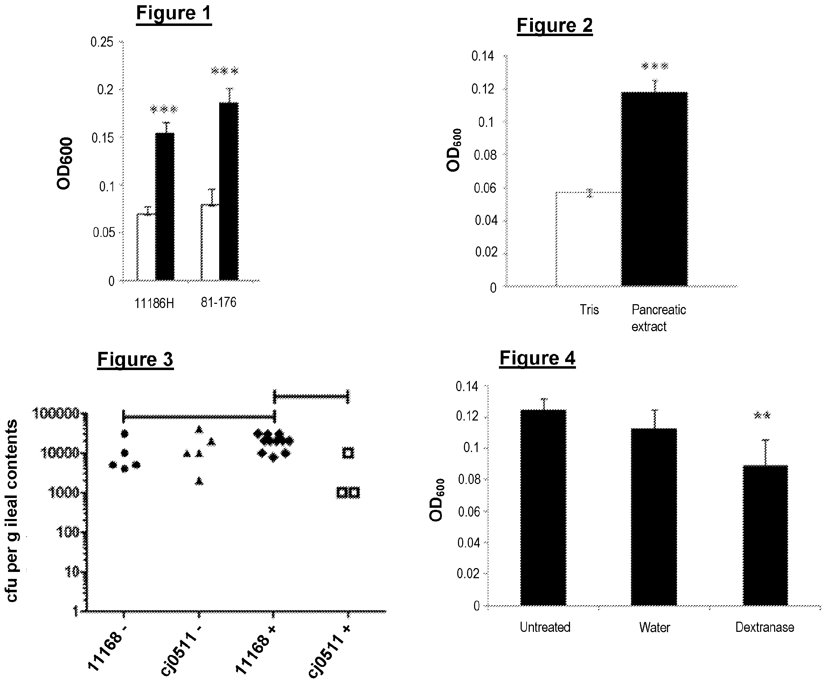

As EPM is an essential component of the biofilm, we measured the ability of the strains to form a biofilm at the air/liquid interface in glass test tubes in the presence and absence of α-amylase. Biofilm was quantified by crystal violet staining as previously described. All the strains, with the exception of the *cj0511* mutant, showed a statistically significant increase in biofilm formation in the presence of pancreatic α-amylase (Figure 1). The magnitude of the increase in biomass in response to amylase in the *waaC, waaF,* and *kpsM* mutants was the same as the wild-type strain (11168H) demonstrating that biofilm formation induced by pancreatic amylase is independent of both LOS and capsule. Since biosynthesis of the CFW-reactive polysaccharide was shown by Mclennan et al 2008 to be increased in a spoT mutant, we constructed a *spoT* mutant and measured biofilm formation in this strain. An increase in biofilm biomass of similar magnitude to the wild-type strain occurred in the *spoT* mutant in response to pancreatic α-amylase indicating that the response is independent of the CFW-reactive polysaccharide. Interestingly, although the *cj0511* mutant displayed increased biofilm formation compared to the wild-type strain in the absence of amylase, there was no increase in biofilm mass in response to the presence of amylase, demonstrating that a functional Cj0511 protease is required for the response to amylase.

Although the primary sequences of pancreatic amylases between mammals and birds are 80% identical, to confirm that chicken pancreatic amylase was also able to induce the response, we prepared a crude chicken pancreas extract and added it to the biofilm assay in place of purified HP amylase. This resulted in an increase in biofilm formation of similar magnitude (2.2-fold, p<0.0001) as the increase apparent with the commercial purified hog amylase preparation (Figure 2). Similarly, an increase in autoagglutination was observed with the crude pancreatic extract that was of similar magnitude to the increase apparent with the purified commercial preparation.

Confocal laser scanning microscopy (CLSM) with live-dead stain was used to visualise the bacteria within an undisturbed biofilm. The 3D confocal images were digitally manipulated using ImageJ software. Comparison of biofilms formed for 48 h on glass coverslips in Mueller-Hinton broth with and without 100 nM HP amylase showed that, whereas in the absence of amylase there are few bacteria adherent to the glass surface, in the presence of amylase there is evidence of typical biofilm structure with adherent microcolonies of live bacteria separated by dark, presumably water-filled, channels. Examination of the biofilms in the x-z plane showed evidence of a three-dimensional structure of consistent depth of 160 µm only in the presence of amylase.

### Example 3 - the EPM promotes resistance to environmental stresses.

In this Example we show the dextran-containing EPM rendered the bacterium more resistant to environmental stresses.

Furthermore induction of EPM led to a an increase in interaction with eukaryotic cell lines and a dramatic increase in killing in the *Galleria* infection model and to an increase in the colonisation of chicks.

### EPM promotes survival in aerobic conditions, at high and low temperatures and at low pH in vitro

To determine the effect of the EPM on resistance to environmental stress, *C. jejuni was* cultured in the absence or presence of 100 nM HP amylase and its resistance to high (65°C) and low temperature (4°C), ambient conditions (20°C in air) and low pH (pH 4.0) was determined. From a starting inoculum of approximately 10⁹, bacteria grown in the presence of amylase were detectable after 9 minutes at 65°C compared to bacteria grown without amylase, counts of which fell below the limit of detection after 6 minutes. At 4°C bacteria grown in the presence of amylase were still detectable after 16 days whereas bacteria grown without amylase were undetectable by day 14. Pre-exposure to amylase also resulted in an increased ability to survive under ambient conditions and at pH 4.0.

### Induction of EPM promotes adhesion and invasion of caco2 cells

To determine if induction of the EPM by pre-exposure to alpha-amylase affects the interaction with and invasion of host epithelial cells, *C. jejuni* strains, grown on MHA with or without 100 µM HP amylase, were co-cultured with Caco-2 cells at a multiplicity of infection (MOI) of 100:1 for 3, 6 or 24 h. Growth on amylase resulted in a highly significant increase in the total numbers of interacting bacteria for both wild-type strains, 11168H and 81-176, at all time points (p<0.0001) with the maximum increase in interaction apparent at 3 h (2.6-3.0 fold). In contrast, exposure to amylase did not produce an increase in the number of interacting *cj0511* mutant bacteria, whereas the complemented strain showed significant increases at all time points (1.5-2.9-fold; p<0.0001). For caco-2 invasion assays, growth on amylase resulted in ∼1.3-fold increased invasion of both wild-type strains at all time points. In contrast, there was no increase in invasion of the cj0511 mutant with pre-exposure to amylase whereas the complemented strain showed a highly significant increase (p<0.0001).

### Induction of the EPM promotes silent translocation through T84 cells

To further explore the role of EPM in interaction with IECs, we measured interleukin-8 (IL-8) secretion from T84 human colon cancer cells in response to bacteria grown with or without HP amylase. At 24 h, a significant increase in IL-8 secretion on stimulation with C. *jejuni* was detected, however there was no difference in IL-8 secretion in response to bacteria grown with or without amylase. Similar results were obtained in THP-1 macrophages: a significant increase on stimulation with the bacteria but no further increase when the EPM was induced. Transepithelial electrical resistance (TEER) measurements in T84 cells showed no disruption of the tight junctions by exposure to C. *jejuni* however there was a significant increase in translocation of the bacteria pre-exposed to amylase compared to those grown without amylase after 6 h, a difference no longer apparent at 24 h.

### Induction of the EPM results in increased killing of Galleria mellonella larvae

To determine whether growth in the presence of alpha-amylase affects potential virulence, the *Galleria* infection model was used (Champion et al., 2010). Strains 11168H, 81-176, and the *cj0511* and *kpsM* mutants were grown with or without HP amylase, inoculated into ten larvae each and the kill determined at 24 h. For both wild-type strains, 1 or 2 larvae were killed when the bacteria were grown without amylase compared to 9 out of 10 larvae when grown with amylase. The increased virulence in response to amylase in the wild-type was lost in the *cj0511* mutant but restored in the complemented strain confirming the essential role of *Cj0511* in signal detection.

### Induction of EPM promotes colonisation in broiler chickens

To determine whether the EPM affects the ability of *C. jejuni* to colonise chickens, strain 11168H and the *cj0511* mutant grown with or without HP amylase were used to infect 21 day old broiler chickens. No differences were apparent at day 4, but by day 7, exposure of the wild type strain to amylase prior to infection had a significant effect (p=0.005) with 11/15 birds colonised when 11168H was grown on amylase compared to 5/15 when grown without amylase (Figure 3). In chickens infected with strains grown in the presence of amylase, the wild type strain was present in significantly higher numbers in the ileum compared to the *cj0511* mutant at days 4 and 7 (p=0.03). This data shows that when the bacterium is covered in the dextran it is better able to colonise the chicken intestine. Thus breakdown of the dextran should reduce colonisation.

### Example 4 - commercially available fungal dextranase disrupts a C. jejuni biofilm

As shown in Figure 4, commercially available dextranase from *Penicillium* sp. disrupts a *C. jejuni* 11168H biofilm.

Additionally, we have inoculated *C. jejuni* 11168H into chickens, recovered the strain and shown using a plate assay that dextranase has activity on the dextran induced in the chicken intestine.

We have also shown by dextranase plate assay that dextranase has activity against five other *C. jejuni* isolates recently obtained from chicken intestine.

### References

Adegbola, R. A., Alabi, S. A., Akinkuade, F. O., Coker, A. O., and Odugbemi, T. (1990). Correlation between human and animal bio-serogroups of Campylobacter isolates in Nigeria. J Trop Med Hyg. 93, 280-283.
Allos, B. M. (2001). Campylobacterjejuni infections: update on emerging issues and trends. Clin. Infect. Dis. 32, 1201-1206.
Bacon, D. J., Szymanski, C. M., Burr, D. H., Silver, R. P., Alm, R. A., and Guerry, P. (2001). A phase-variable capsule is involved in virulence of Campylobacter jejuni 81-176. Mol Microbiol. 40, 769-77.
Beery, J. T., Hugdahl, M. B., and Doyle, M. P. (1988). Colonization of gastrointestinal tracts of chicks by Campylobacterjejuni. Appl. Environ. Microbiol.54, 2365-2370.
Bernatchez, S., Szymanski, C. M., Ishiyama, N., Li, J., Jarrell, H. C., Lau, P. C., Beghuis, A. M., Young, N. M., and Wakarchuk, W. W. (2005). A single bifunctional UDP-GlcNAc/Glc 4-epimerase supports the synthesis of three cell surface glycoconjugates in Campylobacterjejuni. J Biol Chem. 280, 4792-802.
Buswell, C. M., Herlihy, Y. M., Lawrence, L. M., McGuiggan, J. T., Marsh, P. D., Keevil, C. W., and Leach, S. A. (1998). Extended survival and persistence of Campylobacter spp. in water and aquatic biofilms and their detection by immunofluorescent-antibody and - rRNA staining. Appl. Environ. Microbiol. 64, 733-741.
Centers for Disease Control and Prevention, 2013. http://www.cdc.gov/features/dsfoodnet2012/reportcard.html
Champion, O. L., Karlyshev, A. V., Senior, N. J., Woodward, M., La Ragione, R., Howard, S. L., Wren, B. W., and Titball, R. W. (2010). Insect infection model for Campylobacter jejuni reveals that O-methyl phosphoramidate has insecticidal activity. J. Inf. Dis. 201, 776-782.
Cogan, T. A., Thomas, A. O., Rees, L. E., Taylor, A. H., Jepson, M. A., Williams, P. H., Ketley, J., Humphrey, T. J. (2007). Norepinephrine increases the pathogenic potential of Campylobacterjejuni. Gut. 56, 1060-5.
Coker, A. O., Isokpehi, R. D., Thomas, B. N., Amisu, K. O., and Obi, C. L. (2002). Human Campylobaeteriosis in developing countries. Emerg Infect. Dis. 8, 237-44.
Connerton, P. L., Timms, A. R., and Connerton, I. F. (2011). Campylobacter bacteriophages and bacteriophage therapy. J. Appl. Microbiol. 111:255-65.
Davey, M. E., and O'Toole, G. A. (2000) Microbial biofilms: from ecology to molecular genetics. Microbiol. Mol. Biol. Rev. 64, 847-867.
Donlan, R. M., and Costerton, J. W. (2002). Biofilms: survival mechanisms of clinically relevant microorganisms. Clin. Microbiol. Rev. 15, 167-193.
Dubois, M., Gilles, K. A., Hamilton, J. K., Rebers, P. A., and Smith, F. (1956). Colorimetric method for determination of sugars and related substances. Anal. Chem. 28, 350-356.
Edwards, L. A., Nistala, K., Mills, D. C., Stephenson, H. N., Zilbauer, M., Wren, B. W., Dorrell, N., Lindley, K. J., Wedderburn, L. R., and Bajaj-Elliott, M. (2010). Delineation of the innate and adaptive T-cell immune outcome in the human host in response to Campylobacter jejuni infection. PLOS One 5, e15398.
Elmi, A., Watson, E., Sandu, P., Gundogdu, O., Mills, D. C., Inglis, N. F., Manson, E., Imrie, L., Bajaj-Elliott, M., Wren, B. W., Smith, D. G., and Dorrell, N. (2012). Campylobacterjejuni outer membrane vesicles play an important role in bacterial interactions with human intestinal epithelial cells. Infect Immun. 80, 4089-98.
European Food Safety Authority and European Centre for Disease Prevention and Control (2013). The European Union Summary Report on Trends and Sources of Zoonoses, Zoonotic Agents and Food-borne Outbreaks in 2011. EFSA Journal 11, 3129. FAO/WHO (2009): Risk assessment of *Campylobacter* spp. in broiler chickens: Technical Report. In Microbiological risk assessment series No12. Geneva.:Food and Agriculture organization of the United Nations/World health organization; 2009:132.
Garin, B., Gouali, M., Wouafo, M., Perchec, A. M., Pham, M. T., Ravaonindrina, N., Urbès, F., Gay, M., Diawara, A., Leclercq, A., Rocourt, J., and Pouillot, R. (2012). Prevalence, quantification and antimicrobial resistance of Campylobacter spp. on chicken neck-skins at points of slaughter in 5 major cities located on 4 continents. Int. J. Food Microbiol. 157, 102-7.
Gaskin, D. J. H., Van Vliet, A. H. M., and Pearson, B. M. (2007). The Campylobacter genetic toolbox: development of tractable and generally applicable genetic techniques for Campylobacterjejuni. Zoon Publ Health 54 (Suppl. 1), 101.
Gerwig, G. J., Kamerling, J. P., Vliegenthart, J. F. G. (1978). Determination of the D and L configuration of neutral monosaccharides by high-resolution capillary g.l.c. Carbohyd. Res. 62, 349-357.
Gillespie, I. A., O'Brien, S. J., Frost, J. A., Adak, G. K., Horby, P., Swan, A. V., Painter, M. J. And Neal, K. R. (2002) A case-case comparison of Campylobacter coli and Campylobacterjejuni infection: a tool for generating hypotheses. Emerg. Infect. Dis. 8, 937-942.
Guerry, P. And Szymanski, C. M. (2008). Campylobacter sugars sticking out. Trends Microbiol. 16, 429-435.
Haddock, G., Mullin, M., MacCallum, A., Sherry, A., Tetley, L., Watson, E., Dagleish, M., Smith, D. G., Everest, P. (2010). Campylobacterjejuni 81-176 forms distinct microcolonies on in vitro-infected human small intestinal tissue prior to biofilm formation. Microbiology. 156, 3079-84.
Humphrey, T., O'Brien, S., and Madsen, M. (2007) Campylobacters as zoonotic pathogens: a food production perspective. Int. J. Food Microbiol. 117, 237-257.
Janssen, R., Krogfelt,K.A., Cawthraw, S. A., vanPelt, W., Wagenaar, J.A.,and Owen, R.J. (2008). Host-pathogen interactions in Campylobaeter infections: the host perspective. Clin. Microbiol. Rev. 21, 505-518.
Joshua, G. W., Guthrie-Irons, C., Karlyshev, A. V., and Wren, B. W. (2006). Biofilm formation in Campylobacter jejuni. Microbiol. 152, 387-396.
Kalmokoff, M., Lanthier, P., Tremblay, T. L., Foss, M., Lau, P. C., Sanders, G., Austin, J., Kelly, J., and Szymanski. (2006). Proteomic analysis of Campylobacter jejuni 11168 biofilms reveals a role for the motility complex in biofilm formation. J. Bacteriol. 188, 4312-4320.
Kanipes, M. I., Papp-Szabo, E., Guerry, P., and Monteiro, M. A. (2006). Mutation of waaC, encoding heptosyltransferase I in Campylobacter jejuni 81-176, affects the structure of both lipooligosaccharide and capsular carbohydrate. J Bacteriol. 188, 3273-9.
Karlyshev, A. V., Linton, D., Gregson, N. A., Lastovica, A. J., and Wren, B. W. (2000). Genetic and biochemical evidence of a Campylobacter jejuni capsular polysaccharide that accounts for Penner serotype specificity. Mol. Microbiol. 35, 529-541.
Khandavilli, S., Homer, K. A., Yuste, J., Basavanna, S., Mitchell, T., and Brown, J. S. (2008). Maturation of Streptococcus pneumoniae lipoproteins by a type II signal peptidase is required for ABC transporter function and full virulence. Mol. Microbiol. 67, 541-557.
Laws, A.P., Chadha, M. J., Chacon-Romero, M., Marshall V. M., Maqsood, M. (2008). Determination of the structure and molecular weights of the exopolysaccharide produced by Lactobacillus acidophilus 5e2 when grown on different carbon feeds Carbohyd. Res. 343, 301-307.
Layton, S. L., Morgan, M. J., Cole, K., Kwon, Y. M., Donoghue, D. J., Hargis, B. M. Pumford, N. R. (2011). Evaluation of Salmonella-vectored Campylobacterpeptide epitopes for reduction of Campylobacter jejuni in broiler chickens. Clin. Vaccine Immunol. 18:449-54.
Madhusudhan, K. T., Mokady, S., and Cogan, U. (1987). Chicken pancreatic enzymes for clinical use: autoactivation of the proteolytic zymogens. J. Sci. Food Agric. 41, 187-193.
Malik-Kale, P., Parker, C. T., and Konkel, M. E. (2008). Culture of Campylobacter jejuni with sodium deoxycholate induces virulence gene expression. J. Bacteriol. 190, 2286-97.
McLennan, M. K., Ringoir, D. D., Frirdich, E., Svensson, S. L. Wells, D. H., Jarrell, H., Szymanski, C. M., and Gaynor, E. C. (2008). Campylobacter jejuni biofilms up-regulated in the absence of the stringent response utilize a calcofluor white-reactive polysaccharide. J. Bacteriol. 190, 1097-1107.
Miller, C. E., Rock, J. D., Ridley, K. A., Williams, P. H., and Ketley, J. M. (2008). Utilization of lactoferrin-bound and transferrin-bound iron by Campylobacter jejuni. J. Bacteriol. 190, 1900-1911.
Mills, D. C., Gundogdu, O., Elmi, A., Bajaj-Elliott, M., Taylor, P. W., Wren, B. W., and Dorrell, N. (2012). Increase in Campylobacter jejuni invasion of intestinal epithelial cells under low-oxygen coculture conditions that reflect the in vivo environment. Infect. Immun. 80, 1690-8.
Misawa, N., and Blaser, M. J. (2000). Detection and characterization of autoagglutination activity by Campylobacter jejuni. Infect. Immun. 68, 6168-6175.
Moe, K. K., Mimura, J., Ohnishi, T., Wake, T., Yamazaki, W., Nakai, M., and Misawa, N. (2010). The mode of biofilm formation on smooth surfaces by Campylobacter jejuni. J. Vet. Med. Sci. 72, 411-416.
Murphy, C., Carroll, C., and Jordan, K. N. (2006). Environmental survival mechanisms of the foodborne pathogen Campylobacter jejuni. J. Appl. Microbiol. 100, 623-632.
Papp-Szabó, E., Kanipes, M. I., Guerry, P., and Monteiro, M. A. (2005). Cell-surface alpha-glucan in Campylobacterjejuni 81-176. Carbohydrate Res. 340, 2218.
de Perio, M. A., Niemeie, R. T., Levine, S. J., Gruszynski, K., and Gibbins, J.D. Campylobacter infection in poultry-processing workers, Virginia, USA, 2008-2011. Emerg Infect Dis [Internet]. 2013 Feb [date cited]. http://dx.doi.org/10.3201/eid1902.121147
Prokhorova T. A., Nielsen, P. N., Petersen, J., Kofoed, T., Crawford, J. S., Morsczeck, C., Boysen, A., and Schrotz-King, P. (2006). Novel surface polypeptides of Campylobacter jejuni as traveller's diarrhoea vaccine candidates discovered by proteomics. Vaccine. 24, 6446-55.
Reeser, R. J., Medler, R. T., Billington, S. J., Jost, B. H., and Joens, L. A. (2007). Characterization of Campylobacter jejuni biofilms under defined growth conditions. Appl. Environ. Microbiol. 73, 1908-1913.
Reuter, M., Mallett, A., Pearson, B. M., and van Vliet, A. H. (2010). Biofilm formation by Campylobacter jejuni is increased under aerobic conditions. Appl. Environ. Microbiol. 76, 2122-8.
Sanders, S. Q., Boothe, D. H., Frank, J. F., and Arnold, J. W. (2007). Culture and detection of Campylobacterjejuni within mixed microbial populations of biofilms on stainless steel. J. Food Prot. 70, 1379-1385.
Sanders, S. Q., Frank, J. F., and Arnold, J. W. (2008). Temperature and nutrient effects on Campylobacterjejuni attachment on multispecies biofilms on stainless steel. J. Food Prot. 71, 271-278.
Slaughter, S. L., Ellis, P. R., and Butterworth, P. J. (2001). An investigation of the action of porcine pancreatic a-amylase on native and gelatinised starches. Biochim. Biophys. Acta 1525, 29-36.
Stellner, K., Saito, H., Hakomori, S. I. (1973). Determination of aminosugar linkages in glycolipids by methylation. Aminosugar linkages of ceramide pentasaccharides of rabbit erythrocytes and of Forssman antigen. Arch. Biochem. Biophys. 155, 464-472.
Trachoo, N., Frank, J. F., and Stern, N. J. (2002). Survival of Campylobacter jejuni in biofilms isolated from chicken houses. J. Food Prot. 65, 1110-1116.
Van Vliet, A. H. M., Wood, A. C., Henderson, J., Wooldridge, K., and Ketley, J. M. (1998). Genetic manipulation of enteric Campylobacter species. Methods Microbiol. 27, 407-419.
Wilson, D. J., Gabriel, E., Leatherbarrow, A. J., Cheesbrough, J., Gee, S., Bolton, E., Fox, A., Fearnhead, P., Hart, C. A., Diggle, P. J. (2008). Tracing the source of Campylobacteriosis. PLoS Genet. 4, e1000203.
Zimmer, M., Barnhart, H., Idris, U., and Lee, M. D. (2003). Detection of Campylobacter jejuni strains in the water lines of a commercial broiler house and their relationship to the strains that colonized the chickens. Avian Dis. 47, 101-107.

## Claims

1. A composition comprising dextranase for use in a method for inhibiting colonization of an animal by *Campylobacter,* said method comprising the steps of administering orally to said animal said composition comprising the dextranase.

2. A composition for use as claimed in claim 1 wherein the method is used to eradicate or reduce the prevalence of *Campylobacter* in a population of animals.

3. A composition for use as claimed in claim 1 wherein the method is for treating *Campylobacter* infection in the animal.

4. A composition for use as claimed in any one of claims 1 to 3 wherein the dextranase is provided in protected form in the composition, wherein the dextranase is optionally present in immobilised form in the core of an enzyme microgranule which has an enteric coating

5. A composition for use as claimed in any one of claims 1 to 4 wherein the dextranase is administered in combination with an animal feed, wherein the dextranase is optionally intimately mixed with the feed.

6. A composition for use as claimed in claim 5 wherein the feed does not contain another antimicrobial drug in addition to the dextranase, or wherein the feed contains another antimicrobial drug in addition to the dextranase, but the other drug is at a concentration that is not effective for treatment and/or prophylaxis of Campylobacter infection in the animal, in the absence of the dextranase.

7. A composition for use as claimed in any one of claims 1 to 6 wherein the animal is a poultry animal, wherein the poultry animal is optionally a chicken.

8. A composition for use as claimed in claim 7 wherein the composition is:
(i) administered from between and 1 day and 4 weeks post hatching, and\or
(ii) is fed to the poultry animal in an amount of about 0.0001 to about 10 grams of dextranase per kg of feed, more preferably about 0.001 to about 1 gram of dextranase per kg of the feed, most preferably about 0.01 to about 0.1 gram of dextranase per kg of the feed, wherein the feed optionally comprises a cereal, which is optionally wheat.

9. A composition for use as claimed in any one of claims 1 to 3 wherein the dextranase is administered in the drinking water for said animal, optionally in the range from 0.001 U to 1000 U/ml, preferably from 0.01 U/ml to 500 U/ml, especially from 0.1 U/ml to 100 U/ml.

10. An *ex vivo* method of inhibiting the virulence or growth, or reducing the number, of *Campylobacter* bacteria in an environment, said method comprising the step of contacting the bacteria or the environment with a composition comprising dextranase.

11. An *ex vivo* method of disrupting a *Campylobacter* bacterial biofilm or inhibiting the formation of a *Campylobacter* bacterial biofilm, said method comprising the step of contacting the biofilm or the bacteria respectively with a composition comprising dextranase.

12. A composition for use or a method as claimed in any one of claims 1 to 11, wherein the *Campylobacter* is *C. jejuni or C. coli.*

13. A composition for use or a method as claimed in any one of claims 1 to 12 wherein the dextranase is a recombinant enzyme and\or a fungal enzyme.

## Patentansprüche

1. Dextranase umfassende Zusammensetzung zur Verwendung in einem Verfahren zur Hemmung der Besiedlung eines Tiers durch Campylobacter, wobei das Verfahren den Schritt des oralen Verabreichens der Dextranase umfassenden Zusammensetzung an das Tier umfasst.

2. Zusammensetzung zur Verwendung nach Anspruch 1, wobei das Verfahren eingesetzt wird, um die Prävalenz von Campylobacter in einer Population von Tieren auszulöschen oder zu verringern.

3. Zusammensetzung zur Verwendung nach Anspruch 1, wobei das Verfahren zur Behandlung einer Campylobacter-Infektion in dem Tier umfasst.

4. Zusammensetzung zur Verwendung nach einem der Ansprüche 1 bis 3, wobei die Dextranase in der Zusammensetzung in geschützter Form bereitgestellt ist, wobei die Dextranase gegebenenfalls in immobilisierter Form im Kern eines Enzym-Mikrogranulats vorliegt, das eine darmlösliche Beschichtung umfasst.

5. Zusammensetzung zur Verwendung nach einem der Ansprüche 1 bis 4, wobei die Dextranase in Kombination mit einem Tierfutter verabreicht wird, wobei die Dextranase gegebenenfalls innig mit dem Futter vermischt ist.

6. Zusammensetzung zur Verwendung nach Anspruch 5, wobei das Futter kein anderes antimikrobielles Arzneimittel zusätzlich zur Dextranase umfasst oder wobei das Futter ein anderes antimikrobielles Arzneimittel zusätzlich zur Dextranase umfasst, wobei das andere Arzneimittel jedoch in einer Konzentration vorliegt, die in Abwesenheit der Dextranase nicht wirksam zur Behandlung und/oder Prophylaxe einer Campylobacter-Infektion in dem Tier ist.

7. Zusammensetzung zur Verwendung nach einem der Ansprüche 1 bis 6, wobei das Tier ein Geflügeltier ist, wobei das Geflügeltier gegebenenfalls ein Huhn ist.

8. Zusammensetzung zur Verwendung nach Anspruch 7, wobei die Zusammensetzung:
(i) zwischen 1 Tag und 4 Wochen nach dem Schlüpfen verabreicht wird und/oder
(ii) dem Geflügeltier in einer Menge von etwa 0,0001 bis etwa 10 Gramm Dextranase pro kg Futter, noch bevorzugter etwa 0,001 bis etwa 1 Gramm Dextranase pro kg Futter, insbesondere etwa 0,01 bis etwa 0,1 Gramm Dextranase pro kg Futter, verabreicht wird, wobei das Futter gegebenenfalls ein Getreide umfasst, das gegebenenfalls Weizen ist.

9. Zusammensetzung zur Verwendung nach einem der Ansprüche 1 bis 3, wobei die Dextranase im Trinkwasser des Tiers verabreicht wird, gegebenenfalls in einer Menge von 0,001 U bis 1000 U/ml, vorzugsweise 0,01 U/ml bis 500 U/ml, insbesondere 0,1 U/ml bis 100 U/ml.

10. Ex-vivo-Verfahren zur Hemmung der Virulenz oder des Wachstums von oder zur Reduktion der Anzahl von Campylobacter-Bakterien in einer Umgebung, wobei das Verfahren den Schritt des Inkontaktbringens der Bakterien oder der Umgebung mit einer Dextranase umfassenden Zusammensetzung umfasst.

11. Ex-vivo-Verfahren zur Zerstörung eines bakteriellen Biofilms von Campylobacter oder zur Hemmung der Bildung eines bakteriellen Biofilms von Campylobacter, wobei das Verfahren den Schritt des Inkontaktbringens des Biofilms bzw. der Bakterien mit einer Dextranase umfassenden Zusammensetzung umfasst.

12. Zusammensetzung zur Verwendung oder Verfahren nach einem der Ansprüche 1 bis 11, wobei das Campylobacter C. jejuni oder C. coli ist.

13. Zusammensetzung zur Verwendung oder Verfahren nach einem der Ansprüche 1 bis 12, wobei die Dextranase ein rekombinantes Enzym und/oder ein Pilzenzym ist.

## Revendications

1. Composition comprenant de la dextranase à utiliser dans un procédé pour inhiber la colonisation d'un animal par *Campylobacter,* ledit procédé comprenant les étapes consistant à administrer par voie orale audit animal ladite composition comprenant de la dextranase.

2. Composition à utiliser selon la revendication 1, dans laquelle le procédé est utilisé pour éradiquer ou réduire la prévalence de *Campylobacter* dans une population d'animaux.

3. Composition à utiliser selon la revendication 1, dans laquelle le procédé est destiné à traiter une infection par *Campylobacter* chez l'animal.

4. Composition à utiliser selon l'une quelconque des revendications 1 à 3, dans laquelle la dextranase est fournie sous une forme protégée dans la composition, dans laquelle la dextranase est facultativement présente sous une forme immobilisée dans le noyau d'un microgranule enzymatique qui présente un enrobage entérique.

5. Composition à utiliser selon l'une quelconque des revendications 1 à 4, dans laquelle la dextranase est administrée en association avec un aliment pour animaux, dans laquelle la dextranase est facultativement mélangée intimement avec l'aliment.

6. Composition à utiliser selon la revendication 5, dans laquelle l'aliment ne contient pas d'autre médicament antimicrobien en plus de la dextranase, ou dans lequel l'alimentation contient un autre médicament antimicrobien en plus de la dextranase, mais l'autre médicament est à une concentration qui n'est pas efficace pour le traitement et/ou la prophylaxie de l'infection par *Campylobacter* chez l'animal, en l'absence de la dextranase.

7. Composition à utiliser selon l'une quelconque des revendications 1 à 6, dans laquelle l'animal est une volaille, dans lequel la volaille est facultativement un poulet.

8. Composition à utiliser selon la revendication 7, dans laquelle la composition est :
(i) administrée entre 1 jour et 4 semaines après l'éclosion, et/ou
(ii) est fournie à la volaille en une quantité d'environ 0,0001 à environ 10 grammes de dextranase par kg d'aliment, de manière plus préférée d'environ 0,001 à environ 1 gramme de dextranase par kg d'aliment, de la manière la plus préférée d'environ 0,01 à environ 0,1 gramme de dextranase par kg de l'aliment, dans laquelle l'aliment comprend facultativement une céréale, qui est facultativement du blé.

9. Composition à utiliser selon l'une quelconque des revendications 1 à 3, dans laquelle la dextranase est administrée dans de l'eau potable pour ledit animal, facultativement dans la plage de 0,001 U à 1000 U/ml, de préférence de 0,01 U/ml à 500 U/ml, en particulier de 0,1 U/ml à 100 U/ml.

10. Procédé *ex vivo* consistant à inhiber la virulence ou la croissance, ou à réduire le nombre, de bactéries du genre *Campylobacter* dans un environnement, ledit procédé comprenant l'étape consistant à mettre en contact les bactéries ou l'environnement avec une composition comprenant de la dextranase.

11. Procédé *ex vivo* consistant à perturber un biofilm bactérien de *Campylobacter* ou à inhiber la formation d'un biofilm bactérien de *Campylobacter,* ledit procédé comprenant l'étape de mise en contact du biofilm ou des bactéries respectivement avec une composition comprenant de la dextranase.

12. Composition à utiliser ou procédé selon l'une quelconque des revendications 1 à 11, où le *Campylobacter* est *C*. *jejuni* ou *C.coli.*

13. Composition à utiliser ou procédé selon l'une quelconque des revendications 1 à 12, dans laquelle la dextranase est une enzyme recombinante et/ou une enzyme fongique.
